(19) Europäisches Patentamt
European Patent Office
Office européen des brevets

(11) Publication number: 0 326 873 A2

# (12) EUROPEAN PATENT APPLICATION

(21) Application number: 89100952.4

(51) Int. Cl.⁴: C07K 9/00

(22) Date of filing: 20.01.89

Claims for the following Contracting States: ES + GR.

(30) Priority: 02.02.88 GB 8802211

(43) Date of publication of application: 09.08.89 Bulletin 89/32

(84) Designated Contracting States: AT BE CH DE ES FR GB GR IT LI LU NL SE

(71) Applicant: GRUPPO LEPETIT S.P.A.
23, Via G. Murat
I-20159 Milano(IT)

(72) Inventor: Trani, Aldo
11, Via Longhi
I-20137 Milano(IT)
Inventor: Tarzia, Giorgio
3, Vicolo San Clemente
I-37100 Verona(IT)

(74) Representative: Macchetta, Francesco
Gruppo Lepetit S.p.A. Patent and Trademark
Department 34, Via Roberto Lepetit
I-21040 Gerenzano (Varese)(IT)

## (54) Teicoplanin hydrazides.

(57) Novel hydrazides of teicoplanin, with antibiotic activity mainly on gram positive bacteria. The compounds of the invention are prepared by reacting an optionally protected teicoplanin starting material with a suitable hydrazine derivative in the presence of a condensing agent and an organic solvent.

EP 0 326 873 A2

Xerox Copy Centre

## TEICOPLANIN HYDRAZIDES

The present invention relates to new antibiotic substances of formula I

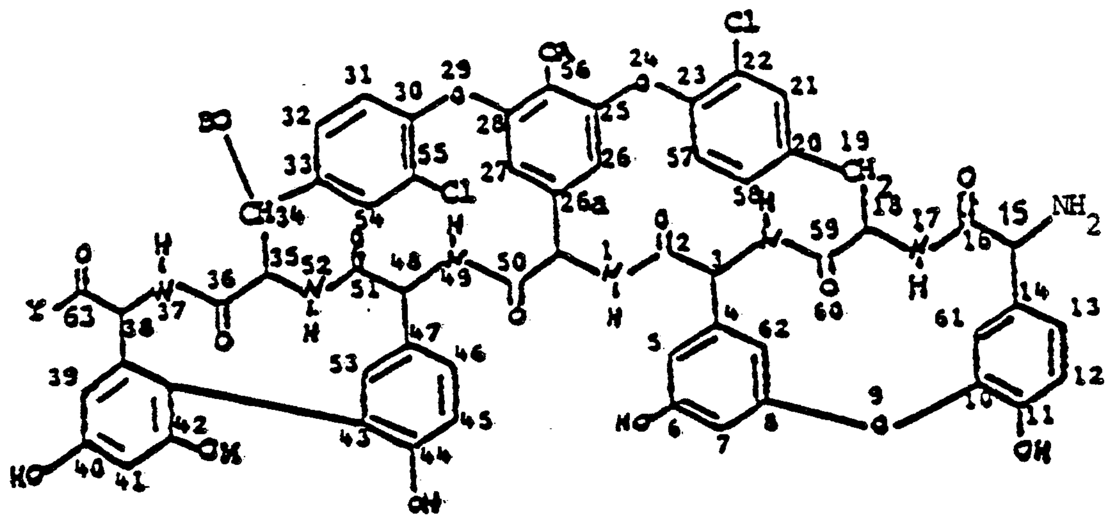

wherein

Y represents a group $R^1R^2N$-NR- wherein

R represents hydrogen or $(C_1$-$C_4)$alkyl,

$R^1$ represents hydrogen, $(C_1$-$C_6)$alkyl, optionally substituted with 1 or 2 groups selected from hydroxy, amino, $(C_1$-$C_4)$alkylamino, di$(C_1$-$C_4)$alkylamino, a nitrogen containing 5-6 membered saturated heterocyclic ring, $(C_1$-$C_4)$alkoxy; $(C_2$-$C_6)$alkenyl, $(C_5$-$C_7)$cycloalkyl, phenyl optionally substituted with a group selected from chloro, bromo, fluoro, iodo, $(C_1$-$C_4)$alkyl and hydroxy,

$R^2$ represents hydrogen or $(C_1$-$C_4)$alkyl, or

$R^1$ and $R^2$ taken together with the adjacent nitrogen atom represent a 5-6 membered saturated heterocyclic ring or a a group of formula $=CR^3R^4$ wherein $R^3$ represents hydrogen or methyl and $R^4$ represents $(C_1$-$C_4)$alkyl or phenyl,

A represents hydrogen or -N[$(C_{10}$-$C_{11})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alfa-D-mannopyranosyl;

with the proviso that B represents hydrogen only when A and M are simultaneously hydrogen and M represents hydrogen only when A is hydrogen and the addition salts thereof.

As used herein the term "alkyl", either alone or in combination with other substituents, includes both straight and branched hydrocarbon groups; more particularly, "$(C_1$-$C_6)$alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexanyl, 2-hexanyl, 3-hexanyl, 3,3-dimethyl-1-butanyl, 4-methyl-1-pentanyl and 3-methyl-1-pentanyl; likewise, "$(C_1$-$C_4)$alkyl" represents a straight or branched hydrocarbon chain of 1 to 4 carbon atoms such as those alkyl of 1 to 4 carbons exemplified above.

The term "$(C_2$-$C_6)$alkenyl" represents straight or branched alkenyl groups of 2, 3, 4, 5 or 6 carbon atoms such as vinyl, allyl, 1 or 2-butenyl, pentenyl, hexenyl, 2-methyl-2-butenyl and the like.

The term "$(C_1$-$C_4)$alkoxy" represents straight or branched alkoxylic groups of 1, 2, 3 or 4 carbon atoms such as methoxy, ethoxy, propoxy, 1-methylethoxy, butoxy and the like.

Examples of nitrogen containing 5-6 membered heterocyclic ring are pyrrolidinyl, piperidinyl, 4-methylpiperidinyl, piperazinyl, 4-methylpiperazinyl, and the like.

Teicoplanin is the international non-proprietary name (INN) of the antibiotic substance formerly named teichomycin which is obtained by cultivating the strain Actinoplanes teichomyceticus nov. sp. ATCC 31121 in a culture medium containing assimilable sources of carbon, nitrogen and inorganic salts (see U.S. Patent No. 4,239,751). According to the procedure described in the above cited patent an antibiotic complex containing Teichomycin $A_1$, $A_2$ and $A_3$ is recovered from the separated fermentation broth by extraction

with a suitable water insoluble organic solvent and precipitation from the extracting solvent according to common procedures. Teichomycin $A_2$, which is the major factor of the isolated antibiotic complex, is then separated from the other factors by means of column chromatography on Sephadex[R]. British Patent Application Publication No. 2121401 discloses that antibiotic Teichomycin $A_2$ actually is a mixture of five closely related co-produced main components.

According to recent structural studies it is possible to represent teicoplanin $A_2$ (formerly Teichomycin $A_2$) main components 1, 2, 3, 4 and 5 by the above formula I wherein R is hydrogen, Y is hydroxy, A represents -N[($C_{10}$-$C_{11}$) aliphatic acyl]-beta-D-2-deoxy-2-amino-glucopyranosyl, B represents N-acetyl-beta-D-2-deoxy-2-amino-glucopyranosyl, M represents alfa-D-mannopyranosyl.

More particularly, in teicoplanin $A_2$ component 1, the [($C_{10}$-$C_{11}$)-aliphatic acyl] substituent represents Z-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties. They are named antibiotic L 17054 and antibiotic L 17046 and are described in European Patent Application Publication No. 119575 and European Patent Application Publication No. 119574, respectively. Preferred hydrolysis conditions for the production of antibiotic L 17054 are: 0.5 N hydrochloric acid at a temperature between 70°C and 90°C and for a time which is generally between 15 and 90 min.

Antibiotic L 17054 is represented by the above formula I wherein Y is hydroxy, R and A represent hydrogen, B represents N-acetyl-beta-D-2-deoxy-2-amino-glucopyranosyl, M represents alfa-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50° and 90°C and for a time which is generally between 30 and 60 min.

Antibiotic L 17046 is represented by the above formula I wherein Y is hydroxy, R, A and M represent hydrogen atoms, and B is N-acetyl-beta-D-2-deoxy-2-amino-glucopyranosyl wherein the sugar moiety is linked to the peptidic nucleus through an O-glycosidic bond.

The complete selective cleavage of all the sugar moieties of the teicoplanin compounds gives an aglycone molecule which is called antibiotic L 17392, or deglucoteicoplanin, and is represented by the above formula I wherein Y is hydroxy, and R, A, B, and M each individually represents a hydrogen group. This selective hydrolysis process is described in European patent application Publication No. 146053.

A substance having the same structural formula is disclosed in European Patent Application Publication No. 0090578 and is named antibiotic A 41030 factor B. This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392 and any mixture thereof in any proportion, are suitable starting materials for the preparation of the hydrazide derivatives of the invention.

In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein R is hydrogen, Y is hydroxy, A represents hydrogen or -N[($C_{10}$-$C_{11}$)aliphatic acyl]-beta-D-2-deoxy-2-amino-glucopyranosyl, B represent hydrogen or N-acetyl-beta-D-2-deoxy-2-amino-glucopyranosyl, M represents hydrogen or alfa-D-mannopyranosyl, with the proviso that B may represent hydrogen only when A and M are simultaneously hydrogen and M may represent hydrogen only when A is hydrogen, a salt thereof, or a mixture thereof in any proportion.

Preferred addition salts of the compounds of this invention are the pharmaceutically acceptable acid addition salts.

With the term "pharmaceutically acceptable acid addition salts", those salts with acids are intended which from biological, manufacturing or formulation standpoint are suitable for use in the pharmaceutical practice as well as in animal growth promotion.

Representative and suitable acid addition salts of the compounds of formula I include those salts formed by standard reaction with both organic and inorganic strong acids such as, for example, hydrochloric, hydrobromic, sulfuric, phosphoric, trifluoroacetic, trichloroacetic, methanesulfonic, benzenesulfonic, picric, benzoic acid and the like.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid addition-salt by dissolving the non-salt form in an aqueous solvent and adding a slight molar excess of the selected acid. The resulting solution or suspension is then lyophilized to recover the desired salt. Instead of lyophilizing, in some instances, it is possible to recover the final salt by extraction with an organic solvent, concentration to a small volume of the separated organic phase and finally precipitation by adding a non-solvent.

In case the final salt is insoluble in an organic solvent where the non-salt form is soluble it is recovered by filtration from the organic solution of the non-salt form after adding the stoichiometric amount or a slight molar excess of the selected acid or base.

The non-salt form can be prepared from a corresponding acid salt dissolved in an aqueous solvent which is then neutralized to free the non-salt form. This is then recovered for instance by extraction with an organic solvent or is transformed into another acid addition salt by adding the selected acid or base and working up as above.

When following the neutralization, desalting is necessary, a common desalting procedure may be employed.

For example, column chromatography on controlled pore polydextrane resins (such as Sephadex L H 20) or silanized silica gel may be conveniently used. After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids or non-pharmaceutically acceptable acids may be used as a convenient purification technique. After formation and isolation, the salt form of a compound of formula I can be transformed into the corresponding non-salt or into a pharmaceutically acceptable salt.

However, in view of the similarity of the properties of the compounds of formula I and their salts, what is said in the present application when dealing with the biological activities of the compounds of formula I applies also to their pharmaceutically acceptable salts, and viceversa.

The compounds of the invention are useful as semi-synthetic antibacterial agents active mainly against gram-positive bacteria, but also active against gram-negative bacteria.

Preferred compounds are those compounds of formula I wherein A, B and M either represent the sugar moieties defined above or represent hydrogen atoms. Other preferred compounds are those wherein the acyl substituent of the sugar defined as substituent A is 8-methylnonanoyl, and B and M are the sugar moieties defined above. Another group of preferred compounds is represented by those compounds of formula I wherein R represents hydrogen or methyl, $R^2$ represents hydrogen or $(C_1$-$C_4)$alkyl and $R^1$ represents benzyl, mono- or di-$(C_1$-$C_4)$alkylamino$(C_1$-$C_4)$alkyl, $(C_1$-$C_4)$alkyl, $(C_5$-$C_6)$cycloalkyl, mono- or di-hydroxy$(C_1$-$C_4)$alkyl, $(C_2$-$C_4)$alkenyl, and amino$(C_1$-$C_4)$alkyl, or $R^1$ and $R^2$ taken together with the adjacent nitrogen atom represent pyrrolidinyl, piperidinyl, 4-methylpiperidinyl, piperazinyl, 4-methylpiperazinyl and morpholinyl. A further group of preferred compounds of the invention is represented by those compounds of formula I wherein A, B and M either represent the sugar moieties defined above or represents hydrogen atom or B and M represent the sugar moieties defined above and A represents the sugar moiety defined above wherein $(C_{10}$-$C_{11})$acyl represents 8-methylnonanoyl and R, $R^1$ and $R^2$ have the meanings reported below:

TABLE I

| R | $R^1$ | $R^2$ |
|---|---|---|
| H | H | H |
| $CH_3$ | H | H |
| $C_2H_5$ | H | H |
| H | $PhCH_2$ | $CH_3$ |
| H | $(CH_3)_2NCH_2CH_2$ | H |
| H | $CH_3CH_2CH_2$ | H |
| H | cyclopentyl | H |
| $CH_3$ | $CH_2CH_2N(CH_3)_2$ | H |
| H | $-CH(CH_3)CH_2N(CH_3)_2$ | H |
| $CH_3$ | $CH_2CH_2NH_2$ | H |
| H | $CH_2CH_2NH_2$ | $CH_3$ |
| H | $CH_2CH_2N$ 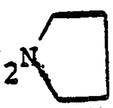 | H |
| H | $CH_2CH_2N$ 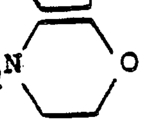 | H |
| H | $CH(CH_3)CH(CH_3)CH_2N(CH_3)_2$ | H |
| H | $CH_2CH_2NH-$ 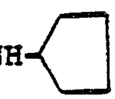 | H |

The compounds of the invention can be prepared by reacting a teicoplanin starting material as above defined with a hydrazine of formula $R^1$ $NR^2$ NHR wherein R $R^1$ and $R^2$ are defined as above, in a polar aprotic solvent in the presence of a condensing agent. In some instances, and in particular when at least one of A, B, and M in the teicoplanin starting material represent hydrogen, it is convenient to protect the primary amino function of the teicoplanin starting material in order to reduce possible undesired side-reactions. This can be done by methods known per se in the art such as those described in reference books like T.W. Greene, "Protective Groups in Organic Synthesis", John Wiley and Sons, New York, 1981, and M. Mc. Omie "Protecting Groups in Organic Chemistry" Plenum Press, New York, 1973. These protecting groups must be stable at the conditions of the reaction process, must not unfavorably interfere with the main reaction, and must be easily cleavable and removable from the reaction medium at the end of the reaction without altering the newly formed hydrazide bond.

Representative examples of N-protecting groups which may be advantageously used in the process of the invention are carbamate forming reagents characterized by the following oxycarbonyl groups: 1,1-dimethylpropynyloxycarbonyl, t-butyloxycarbonyl, vinyloxycarbonyl, aryloxycarbonyl, cinnamyloxycarbonyl, benzyloxycarbonyl, p-nitrobenzyloxycarbonyl-3,4-dimethoxy-6-nitrobenzyloxycarbonyl, 2,4-dichlorobenzyloxycarbonyl, 5-benzisoxazolylmethyloxycarbonyl, 9-anthranylmethyloxycarbonyl, diphenylmethyloxycarbonyl, isonicotinyloxycarbonyl, diphenylmethyloxycarbonyl, isonicotinyloxycarbonyl, S-benzyloxycarbonyl, and the like.

Obviously, when the final compound of formula I contains groups which are labile under acidic

conditions, e.g. when A, B or M represent sugar moieties as above defined which may be hydrolized in an acidic medium, other removal conditions must be used, such as catalytic hydrogenation using for instance Palladium on carbon as the catalyst to remove the proper protecting group. In this case, however, attention should be paid to the presence of groups which may be modified by catalytic hydrogenation. A typical consequence of the catalytic hydrogenation of a derivative of formula I wherein A represents a group as above defined whose acyl portion is Z-decenoyl (i.e. a teicoplanin $A_2$ component 1 derivative or a mixture containing it) is that it is at least partially transformed into the corresponding decanoyl derivative (i.e. a derivative of teicoplanin $A_2$ component 3).

The man skilled in the art is capable, also on the basis of the present disclosure, of deciding which functions need to be protected, how they must be protected and how should they be properly deprotected.

As it is appreciated by the skilled technician, the ultimate choice of the specific protecting group depends on the characteristics of the particular hydrazide derivative which is desired. In fact, this hydrazide function of the final compound should be stable at the condition of removal of the protecting group(s). Since the conditions of removal of the different protecting groups are known, the skilled technician is capable of selecting the proper protecting group. For instance, where the final compound possess also a benzyl ester function or N-benzyl function, the protecting groups which are usually removable by catalytic hydrogenation, such as the benzyloxycarbonyl group, should be avoided, while those protecting groups which are removable under acidic conditions, such as t.butoxycarbonyl, can be conveniently used.

Inert organic solvents useful for this condensation reaction are those organic aprotic solvents which do not unfavorably interfere with the reaction course and are capable of at least partially solubilizing the teicoplanin starting material. Examples of said inert organic solvents are aliphatic amides, alkyl ethers, ethers of glycols and polyols, phosphoramides, sulfoxides, cyclic substituted ureas and aromatic compounds. Preferred examples of inert organic solvents are: dimethylformamide, dimethoxyethane, hexamethylphosphoramide, dimethylsulfoxide, benzene, toluene and mixtures thereof.

The condensing agent in this process is a condensing agent suitable for forming hydrazide bonds in organic compounds.

Representative and preferred examples of these condensing agents are $(C_1-C_4)$alkyl, phenyl or heterocyclic phosphorazidates such as, diphenyl phosphorazidate (DPPA), diethyl phosphorazidate , di(4-nitrophenyl)phosphorazidate, dimorpholylphosphorazidate and diphenylphosphorochloridate. The preferred condensing agent is diphenyl phosphorazidate (DPPA) or diethylphosphoryl cyanide (DEPC).

In the process of the invention, the reactant $R^1R^2NNRH$ is normally used in a molar excess. In general, a 2- to 6-fold molar excess is used while a 3- to 5-fold molar excess is preferred. The condensing agent is generally added in a 2- to 3-molar excess to the teicoplanin starting material. In most instances, it is also necessary to add a base to make the whole reaction medium more basic. In this case, the added base must be such that it does not negatively interfere with the reaction course. Examples of these bases are aliphatic or alicyclic amines such as trimethylamine, triethylamine, N-methyl pyrrolidine or heterocyclic bases such as pycoline, and the like. This base is generally present in a 4 to 6 molar proportion with the starting teicoplanin derivative.

The reaction temperature may vary from 0 and 40-50 °C and conveniently is room temperature. In general, it is preferred to add the reactant at about 0 °C and then to gently rise the temperature to ambient temperature after a certain reaction time (about 30 min). The reaction time varies with the other specific reaction parameters and is generally within 3 and 48 h.

In any case, the reaction course is monitored by TLC or preferably by HPLC according to methods known in the art.

On the basis of the results of these assays, a man skilled in the art will be able to evaluate the reaction course and decide when to stop the reaction and start working up the reaction mass according to known per se techniques which include for instance extraction with solvents, precipitation by non-solvents, in conjunction with further separation and purification by column chromatography.

This method is particularly suitable for preparing those derivatives of formula I wherein R represents hydrogen. In fact, under these reaction conditions, the primary amino group of the hydrazine of formula $R^1R^2NNH_2$ will react to give the corresponding compounds of formula I wherein Y represents $R^1NR^2NH$-.

An alternative method for preparing all the compounds of formula I includes starting from a teicoplanin starting material protected on the 15-amino function and reacting it with an activated ester forming reagent to form an "activated" ester function that can be easily substituted by a hydrazine of formula $R^1NR^2NHR$ to give the corresponding hydrazide of formula I. The amino protected teicoplanin starting material is prepared as known in the art according to the methods described above. The formation of "activated" esters is described in general terms in Fieser and Fieser, Reagents for Organic Synthesis, John Wiley and Sons Inc., 1967, pages 129-130.

Examples of said activated ester forming reagents than can be conveniently used in the process of the invention are those described by R. Schwyzer et al. in Helv. Chim. Acta, 1955, 38, 69-70 and encompass: $ClCH_2CN$, $BrCH_2COOC_2H_5$, $BrCH(COOC_2H_5)_2$, $ClCH_2COCH_3$,

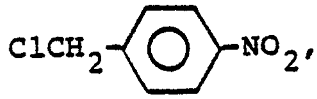

$ClCH_2CH_2N(C_2H_5)_2$.

A preferred reagent of this type is chloroacetonitrile.

The reaction solvent is in this case a polar solvent as defined above. Preferably, this solvent is selected from acetic acid, acetonitrile, dimethylsulfoxide, dimethylformamide and chloroacetonitrile. In some instances, such as when chloroacetonitrile is employed, dimethylformamide is the preferred solvent. The formation of the activated ester is generally conducted in the presence of a base which does not interfere with the reaction course such as a tri-alkylamine like triethylamine, sodium or potassium carbonate or bicarbonate. Generally, the base is employed in a 2 to 6 molar proportion to the teicoplanin starting material and preferably it is used in about a three-fold molar excess. A preferred base is triethylamine.

The activated ester forming reagent is used in a large excess over the teicoplanin starting material. It is in general used in a 5 to 30 molar proportion and preferably, it is used in about a 20-times molar excess. The reaction temperature is between 10°C and 80°C and preferably is about 50°C. As usual, the reaction time depends on the other specific reaction parameters and may be generally between 3 and 48h. Also in this case, the reaction course may be followed by HPLC or TLC to determine when the reaction may be considered as completed and the procedures to recover the desired intermediate can be started. In general, it is isolated by precipitation with non-solvents or extraction with solvents and in general it is used as such in the next reaction step. If desired, however, it may be purified by a column chromatography such as flash column chromatography or reverse phase column chromatography.

The obtained "activated" ester intermediate is then reacted with a molar excess of the hydrazine derivative of formula $R^1NR^2NHR$, preferably in the form of the corresponding acetate, in the presence of a polar solvent as defined above and at a temperature been 10°C and 80°C. Preferred examples of polar solvents are in this case ethanol, dimethylformamide and acetonitrile.

A preferred reaction temperature is about 50°C, while a preferred molar proportion between the intermediate and the hydrazine derivative as above defined is from 1:5 to 1:40, and most preferably is about 1 to 10.

The reaction course may be monitored as usual and the reaction product may be isolated according to known per se techniques.

When R and/or $R^1$ are different from hydrogen, the most nucleophilic nitrogen group of the hydrazine reactant will link to the carbonyl group of the teicoplanin compound to form the hydrazide function.

This is important in particular to direct the reaction process to the formation of the desired compound of formula I. In general, these reactions are specific and only one of the possible products is recovered, however, in case a mixture of products is obtained it may be purified as conventional in the art by, for instance, column chromatography.

It is evident that in many instances a compound of the invention may be prepared in more than one way and also that a compound of the invention may be transformed into another by means of known per se reactions.

For instance, a compound of formula I wherein R is hydrogen or $(C_1\text{-}C_6)$alkyl and $R^1$ or $R^2$ are as above but different from hydrogen may either be obtained according to one of the methods reported above or preferably by reductive alkylation of the compound of formula I wherein $R^1$ and $R^2$ represent hydrogen. In this latter case, the compound of formula I wherein $R^1$ and $R^2$ are both hydrogen atoms (or at least $R^1$ or $R^2$ is hydrogen) is reacted with a suitable carbonyl derivative (aldehyde ketone or a derivative thereof) to form the corresponding Shiff base intermediate which is then reduced with a suitable reducing agent to give the corresponding "alkylated" derivative.

This Schiff base intermediate can in many instances be isolated from the reaction mass or, conveniently reduced without purification to give the desired final compound. The Schiff base formation step is generally conducted in a polar solvent like those defined above. The reaction medium generally has an acidic pH and the temperature is generally between 10°C and 80°C.

Examples of said polar solvents which are preferred in this reaction step are methanol, acetic acid, n-butanol, acetonitrile, the mixtures thereof and their mixtures with water, and dimethylformamide. The acid

medium may be obtained by adding an organic or inorganic acid which essentially acts as an acid catalyst. A preferred acid is p.toluensulfonic acid. The carbonyl compound is an aldehyde or a ketone or a compound capable of giving such a reactive function under the proper reaction conditions, and is generally employed in a large molar excess, its proportion to the teicoplanin compound being between 5:1 and 50:1, and preferably about 20:1.

Once obtained, the Schiff base intermediate can be reduced under conditions that do not alter the teicoplanin nucleus. In case teicoplanin $A_2$ component 1 is not present or it is desired to transform it into the corresponding compound having the saturated N-acyl chain (i.e. a derivative of teicoplanin $A_2$ component 3), the reduction can be a catalytic hydrogenation in the presence of usual hydrogenation catalysts such as transition metals or transition metal derivatives, e.g. oxides, generally on suitable supports such as palladium on barium sulfate, palladium on charcoal, palladium on allumina, palladium on calcium carbonate, platinum, platinum oxide, rhodium on charcoal and the like. A particularly preferred hydrogenation catalyst is palladium on charcoal and most preferably 5% palladium on charcoal. The reaction medium must be compatible with the catalyst as well as the starting material and the final product and is preferably an acidified aqueous alcoholic medium. In some instances however, a slightly basic medium is required by the catalyst, as in the case of a basic support such as allumina or $CaCO_3$. In this case, the reaction medium may conveniently include an alkyl amine like triethylamine, or other non-reactive amines. In addition to 5% palladium on charcoal, 10% palladium on charcoal, 10% palladium on barium sulfate, 5% palladium on calcium carbonate, 5% rhodium on charcoal can also be cited as suitable catalysts.

A preferred acidic aqueous alcoholic medium is represented by a mixture of water and a water soluble lower alkanol, preferably of one to four carbon atoms, in the presence of a mineral acid such as a hydrohalidic acid.

On the contrary, where milder conditions are necessary that do not modify the unsaturation on the acyl

protic solvent selected from aliphatic acids and alpha-halogenated aliphatic acids which at the reaction temperature are liquids, aliphatic and cycloaliphatic alkanols which at the reaction temperature are liquids slightly mixable with water, phenylsubstituted lower alkanols wherein the phenyl moiety may optionally carry $(C_1-C_4)$alkyl, $(C_1-C_4)$alkoxy or halo rests which at the reaction temperature are liquids slightly mixable with water, and beta-polyhalogenated lower alkanols, which at the reaction temperature are liquids; in the presence of a strong acid, compatible with the solvent, selected from strong mineral acids, strong organic acids and strong acid cation exchange resins in the hydrogen form and at a temperature between 20°C and 100°C.

In this case, the preferred hydrolysis conditions are represented by the use of a mineral acid, such as hydrochloric acid, in an haloalkanol such as trifluoroethanol, at a temperature between 65°C and 85°C.

Analogous selective hydrolysis conditions on a similar substrate are described in European Patent Application Publication No. 146053.

Some physico-chemical and biological data of representative compounds of the invention are reported below.

TABLE II

| Compound | A | B | M | R | $R^1$ | $R^2$ |
|---|---|---|---|---|---|---|
| 1 | $-GNHCOR_{(1,5)}$ | $-GNHCOCH_3$ | -M | H | H | H |
| 2 | H | H | H | H | H | H |
| 3 | H | H | H | $CH_3$ | H | H |
| 4 | H | H | H | $C_2H_5$ | H | H |
| 5 | H | H | H | H | $PhCH_2$ | H |
| 6 | H | H | H | H | $CH_3CH_2CH_2$ | H |
| 7 | H | H | H | H | $(CH_3)_2NCH_2CH_2$ | H |
| 8 | $-GNHCOR_{(1,5)}$ | $-GNHCOCH_3$ | -M | H | cyclopentyl | |
| 9 | H | H | H | H | $CH_3CH_2CH_2$ | $CH_3CH_2CH_2$ |
| 10 | H | H | H | H | $CH_3CH_2CH_2$ | H |

TABLE II (continued)

| Compound | A | B | M | R | $R^1$ | $R^2$ |
|---|---|---|---|---|---|---|
| 11 | H | H | H | $CH_3$ | $CH_3$ | $CH_3$ |
| 12 | H | H | H | H | $CH_2OH-CHOH-CH_2$ | H |
| 13 | H | H | H | H | $CH_3-CH=CH-CH=$ | |
| 14 | H | H | H | H | H | cyclopentyl |
| 15 | H | H | H | H | $(CH_3)_2CH-CH_2-CH(CH_3)-$ | H |
| 16 | H | H | H | H | $Ph-C(CH_3)=$ | |
| 17 | H | H | H | H | $H_2NCH_2CH_2$ | H |

Note:

$-GNHCOR_{(1-5)}$ = N/(C$_{10}$-C$_{11}$)aliphatic acyl/-beta-D-2-deoxy-2-aminoglucopyranosyl

$-GNHCOR_{(2,3)}$ = N-(8-methylnonanoyl)-beta-D-2-deoxy-2-aminoglucopyranosyl and N-decanoyl-beta-D-2-deoxy-2-aminoglucopyranosyl

$-GNHCOR_{(4,5)}$ = N-(8-methyldecanoyl)-beta-D-2-deoxy-2-aminoglucopyranosyl and N-(9-methyldecanoyl)-beta-D-2-deoxy-2-aminoglucopyranosyl

$-GNHCOCH_3$ = N-acetyl-beta-D-2-deoxy-2-aminoglucopyranosyl

-M = alfa-D-mannopyranosyl

An example of the way in which the reaction may be monitored by HPLC is as follows: sample of about 50 microliter is drawn from the reaction mixture at a predetermined time, diluted to a final concentration of about 0.5 mg/ml in a mixture 0.2% aqueous sodium dihydrogen fosfate/acetonitrile 50/50 (v/v) and injected into the HPLC system.

The HPLC system may be a chromatograph Hewlett-Packard 1084A equipped with a UV detector at 254 nm and a stainless steel precolumn Hibar LiChroCart packed with LiChrosorb RP-8 Merck Co. (5 micrometer) $C_8$-alkyl silanized silica gel followed by a Hibar LiChroCART column (15 cm) pre-packet with LiChrosorb RP-8 (5 micrometer)

Flow rate: 1.0 ml/mn

Injection volume: 20 microliter

Eluents:

solution A: acetonitrile/sodium dihydrogen fosfate (0.02M) 5/95 (v/v)

solution B: acetonitrile/sodium dihydrogen fosfate (0.02M) 75/25 (v/v)

Gradient profile:

| [1]: | | | | | | |
|---|---|---|---|---|---|---|
| min<br>%B | 0<br>15 | 15<br>35 | 20<br>45 | 25<br>60 | 27<br>15 | 28<br>stop |
| [2]: | | | | | | |
| min<br>%B | 0<br>30 | 2<br>30 | 25<br>50 | 30<br>60 | 35<br>30 | 36<br>stop |

The retention time of the compounds of this invention in the above system is reported in Table III below:

TABLE IIIa

HPLC analysis of Table I compounds

| Compound No. | $t_R$ (min) Gradient Profile | |
|---|---|---|
| | [1] | [2] |
| 1 | 15.3 | |
| 2 | 9.0 | 2.9 |
| 3 | 9.6 | 2.7 |
| 4 | 10.2 | |
| 5 | | 7.8 |
| 6 | 10.5 | |
| 7 | | 6.3 |
| 8 | | 6.4* |
| 9 | | 6.8 |
| 10 | 10.6 | |
| 11 | 8.8 | |
| 12 | | 7.4 |
| 13 | 12.8 | |
| 14 | 13.4 | |
| 15 | | 8.9 |
| 16 | | 7.5 |
| 17 | | 5.3 |

## TABLE IIIb

HPLC retention time ($t_R$) of intermediates

| Intermediate | $t_R$ Gradient profile /2/ |
|---|---|
| V | 18.3* |
| VI | 23.3* |
| VII | 21.6* |
| VIII | 12.3 |
| IX | 23.6 |
| X | 15.6 |
| XI | 17.7 |
| XII | 18.5 |
| XIII | 24.2 |
| XIV | 18.8 |
| XI | 21.3 |
| XVI | 22.9 |
| XVII | 10.4 |
| XVIII | 16.3 |
| XIX | 15.1 |
| XX | 20.8 |
| XXI | 20.5 |
| XXII | 23.2 |
| XXIII | 23.7 |
| XXIV | 20.8 |

* $t_R$ of the highest peak of the complex (i.e. component 2)

The antibacterial activity of the compounds of the invention can be demonstrated in vitro by means of standard agar-dilution tests. Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37°C. The results of the antibacterial testing of representative compounds of formula I are summarized in Table IV below:

TABLE IV

In vitro activity

MIC (mcg/ml)

| Microorganism | Compound numbers 1 | 2 | 3 | 4 | 5 | 6 |
|---|---|---|---|---|---|---|
| Staph.aureus Tour L165 | 0.25 | 0.125 | 0.125 | 0.063 | 0.063 | 0.063 |
| Staph.aureus Tour $10^6$ cfu/ml | 0.0125 | 0.125 | 0.125 | 0.125 | 0.125 | 0.125 |
| Staph.aureus Tour 30% bovine serum | 0.5 | 1 | 0.5 | 0.5 | 1. | 0.5 |
| Staph.epidermidis ATCC 12228 | 0.125 | 0.063 | 0.063 | 0.063 | 0.063 | 0.063 |
| Staph.heamoliticus L602 clin.isolate | 1 | 0.25 | 0.125 | 0.125 | 0.125 | 0.25 |
| Strep.pyogenes L49 C 203 | 0.063 | 0.125 | 0.125 | 0.125 | 0.063 | 0.125 |
| Strep.pneumoniae L44 UC 41 | 0.063 | 0.125 | 0.125 | 0.125 | 0.063 | 0.125 |
| Strep.faecalis ATCC 7080 | 0.125 | 0.125 | 0.125 | 0.125 | 0.125 | 0.125 |
| Haemophilus influenzae type b ATCC 19418 | 128 | 32 | 64 | 64 | 64 | 64 |
| E.coli SKF 12140 | > 128 | 16 | 32 | 32 | > 128 | 32 |
| Strep.mitis L796 | 0.125 | 0.125 | 0.125 | 0.125 | 0.063 | 0.125 |
| Neisseria gonorrhoeae ISM 68/126 | 64 | 64 | 16 | 32 | 32 | 32 |
| Proteus vulgaris X19H ATCC 881 | > 128 | 64 | 128 | 128 | > 128 | > 128 |
| Pseudomonas aeruginosa L4 ATCC 10145 | > 128 | 64 | > 128 | 128 | > 128 | 128 |

TABLE IV (continued)

In vitro activity

MIC (mcg/ml)

| Microorganism | Compound numbers 7 | 8 | 9 | 10 | 11 | 12 |
|---|---|---|---|---|---|---|
| Staph.aureus Tour L165 | 0.063 | 0.25 | 0.125 | 0.063 | 0.125 | 0.125 |
| Staph.aureus Tour $10^6$ cfu/ml | 0.125 | 0.5 | 0.125 | 0.125 | 0.125 | 0.125 |
| Staph.aureus Tour 30% bovine serum | 1 | 1 | 1 | 0.5 | 2 | 1 |
| Staph.epidermidis ATCC 12228 | 0.063 | 0.125 | 0.063 | 0.063 | 0.063 | 0.063 |
| Staph.heamoliticus L602 clin.isolate | 0.125 | 4 | 0.125 | 0.25 | 0.125 | 0.125 |
| Strep.pyogenes L49 C203 | 0.125 | 0.063 | 0.063 | 0.063 | 0.125 | 0.125 |
| Strep.pneumoniae L44 UC41 | 0.125 | 0.125 | 0.125 | 0.125 | 0.125 | 0.125 |
| Strep.faecalis ATCC 7080 | 0.125 | 0.125 | 0.125 | 0.125 | 0.125 | 0.125 |
| Haemophilus influenzae type b ATCC 19418 | | 128 | 64 | 128 | 64 | 64 |
| E.coli SKF 12140 | 8 | > 128 | 32 | 32 | 32 | 32 |
| Strep.mitis L796 | 0.125 | 0.125 | 0.125 | 0.125 | 0.125 | 0.125 |
| Neisseria gonorrhoeae ISM 68/126 | 32 | 32 | 16 | 32 | 64 | 64 |
| Proteus vulgaris X19H ATCC 881 | 32 | > 128 | > 128 | 128 | 128 | 128 |
| Pseudomonas aeruginosa I.4 ATCC 10145 | 32 | > 128 | 128 | > 128 | 128 | 128 |

TABLE IV (continued)

In vitro activity

MIC (mcg/ml)

| Microorganism | Compound numbers 13 | 12 | 15 | 16 |
|---|---|---|---|---|
| Staph.aureus Tour L165 | 0.125 | 0.063 | 0.125 | 0.063 |
| Staph.aureus Tour $10^6$ cfu/ml | 0.125 | 0.125 | 0.125 | 0.125 |
| Staph.aureus Tour 30% bovine serum | 2 | 2 | 2 | 4 |
| Staph.epidermidis ATCC 12228 | 0.063 | 0.063 | 0.06 | 0.63 |
| Staph.heamoliticus L602 clin.isolate | 0.125 | 0.125 | 0.125 | 0.25 |
| Strep.pyogenes L49 C203 | 0.125 | 0.125 | 0.125 | 0.063 |
| Strep.pneumoniae L44 UC41 | 0.125 | 0.125 | 0.125 | 0.063 |
| Strep.faecalis ATCC 7080 | 0.125 | 0.125 | 0.125 | 0.125 |
| Haemophilus influenzae type b ATCC 19418 | 64 | 64 | 32 | 32 |
| E.coli SKF 12140 | 16 | 32 | 32 | 16 |
| Strep.mitis L796 | 0.125 | 0.125 | 0.125 | 0.063 |
| Neisseria gonorrhoeae ISM 68/126 | 64 | 32 | 16 | 16 |
| Proteus vulgaris X19H ATCC 881 | > 64 | > 64 | > 128 | > 128 |
| Pseudomonas aeruginosa L4 ATCC 10145 | > 64 | > 64 | > 128 | > 128 |

The antimicrobial activity of the compounds of the invention is confirmed also in experimental septicemia in the mouse.

Control and treatment groups contain ten CD-1 mice (Charles River) weighing 18-22 g. They are infected intraperitoneally with 0.5 ml of bacterial suspension prepared by diluting an overnight culture of S. pyogenes C 203 (L 49) with sterile peptonized saline. Inocula are adjusted so that untreated animals died of septicemia within 48 h. The compounds to be tested are administered subcutaneously immediately after infection. On the 7th day, the $ED_{50}$ in mg/kg is calculated by the method of Spearman and Kärber (D.J. Finney, "Statistical Methods in Biological Assay", Griffin, page 524, 1952) from the percentage of surviving animals at each dose.

In view of the above reported antimicrobial activity, the compounds of the present invention can effectively be employed as the active ingredient of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion. The compounds of the present invention can be administered orally, topically or parenterally wherein however, the parenteral administration is preferred. Depending on the route of administration, these compounds can be formulated into various dosage forms. Preparations for oral administration may be in the form of capsules, tablets, liquid solutions or suspensions. As known in the art the capsules and tablets may contain in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavoring agents, and acceptable disintegrating and wetting agents. The liquid preparations generally in the form of aqueous or oily solutions or suspensions, may contain conventional additives such as suspending agents. For topical use the compounds of the present invention may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-liquid form. Topical applications may be formulated in hydrophobic or hydrophilic bases as ointments, creams, lotions, paints, or powders.

For rectal administration the compounds of the invention are administered in the form of suppositories admixed with conventional vehicles, such as, for example, cocoa butter, wax, spermaceti or polyethylenglycols and their derivatives.

Compositions for injection may take such forms as suspensions, solutions, or emulsions in oily or aqueous vehicles, and may contain formulatory agents such as suspending, stabilizing and/or dispersing agents.

Alternatively, the active ingredient may be in powder form for reconstitution at the time of delivery with a suitable vehicle, such as sterile water.

The amount of active principle to be administered depends on various factors such as the size and conditions of the subject to be treated, the route and frequency of administration, and the causative agent involved.

The compounds of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from about 20 to about 300 mg per unit.

Representative examples of preparation of pharmaceutical compositions are as follows:

A parenteral solution is prepared with 100 mg of compound No 3 dissolved in 2 ml of sterile water for injection. A parenteral solution is prepared with 250 mg of compound N 19 hydrochloride dissolved in 3 ml of sterile water for injection.

A topical ointment is prepared with 200 mg of compound No 7.

3.6 g of polyethylene glycol 4000 U.S.P.

6.2 g of polyethylene glycol 400 U.S.P.

Besides their activity as medicaments, the compounds of the present invention can be used as animal growth promoters.

For this purpose, one or more of the compounds of the invention is administered orally in a suitable feed. The exact concentration employed is that which is required to provide for the active agent in a growth promotant effective amount when normal amounts of feed are consumed.

The addition of the active compounds of the invention to animal feed is preferably accomplished by preparing an appropriate feed premix containing the active compounds in an effective amount and

incorporating the premix into the complete ration.

Alternatively, an intermediate concentrate or feed supplement containing the active ingredient can be blended into the feed.

The way in which such feed premixes and complete rations can be prepared and administered are described in reference books (such as "Applied Animal Nutrition", W.H. Freedman and Co., S. Francisco, USA, 1969 or "Livestock Feeds and Feeding", O and B Books, Corvallis, Oregon, USA, 1977) and are incorporated herein by reference.

The following examples further illustrate the invention without limiting its scope.

Example 1A: Preparation of teicoplanin $A_2$ hydrazide (compound 1) - Method A

Teicoplanin (1 g; 0.53 meq) is dissolved in DMF (30 ml) and the solution is cooled to 0°C, then in close succession 37% w/w hydrochloric acid (0.05 ml), 98% aqueous hydrazine (0.05 ml), diphenylphosphoryl azide (DDPA; 0.250 ml) and triethylamine (0.130 ml) are added. The reaction mixture is stirred 3 hours and 0°C and then at room temperature overnight. After this time about 10% of the starting material is still detectable by HPLC analysis.

Another portion of DPPA (0.05 ml) is added at room temperature and stirring is continued for further three hours.

The reaction mixture is worked up by pouring it into 250 ml of water; the pH is adjusted to about 4 by adding 1N HCl, then the resulting mixture is extracted with a mixture n-butanol/ethyl acetate, 3:1 (v/v) (200 ml). The organic phase is washed twice with distilled water and then concentrated to about 10 ml under reduced pressure.

By adding ethyl ether (150 ml) a solid separates which is collected, washed with fresh ether and dried under reduced pressure, yielding 700 mg of crude teicoplanin $A_2$ hydrazide.

This product is purified by column chromatography on 150 g of silanized silica-gel (0.06-0.2 mm, Merck Co.), re-equilibrated in water:acetonitrile:acetic acid 90:10:0.1. A solution of the crude product in water:acetonitrile 70:30 is then loaded on the column that is eluted with a linear gradient of $CH_3CN$ in water containing 0.1% $CH_3COOH$, from 20 to 40% using a total of 3 l of eluent mixture. Fractions (20 ml) containing the pure product are pooled and evaporated and the obtained solid, is suspended in ether, filtered, washed with fresh ether and dried in vacuum overnight at 35°C, yielding 100 mg of title compound.

Formula $C_{88-89}$ $H_{97-101}$ $O_{32}$ $N_{11}Cl_2$ Mw about 1899

microanalysis: after drying at 140°C $\Delta$wt 9.5%

| | | | |
|---|---|---|---|
| Calc.: | C 55.95; | H 5.3; | N 8.1 |
| Found: | C 54.05; | H 5.49; | N 7.47 |

Potentiometric titration $pK_{mcs}$ 6.5

Example 1B: Preparation of teicoplanin $A_2$ hydrazide (compound 1) - Method B

a) Preparation of $N^{15}$-CBZ-teicoplanin (intermediate V)

Benzylchloroformate (0.8 ml, 5.6 meq) is added to a solution of teicoplanin (10 g; 5.3 meq) in 300 ml of DMF containing triethylamine (1 ml). The reaction is stirred at room temperature for about one hour. The reaction is checked by HPLC to see when it can be considered as completed. Then, the pH of the reaction mixture is adjusted to about 5 by adding a few drops of TEA and the solvent is removed at 50°C under reduced pressure. The residue is dissolved in water and extracted with n-butanol/ethyl acetate, 1:1 (v/v) (250 ml). The organic phase is washed with distilled water, concentrated to about 10 ml. By diluting with ethyl acetate a solid precipitates that is filtered, washed with ethyl ether and dried under reduced pressure at 35°C, yielding 9.4 g of pure $N^{15}$-CBZ teicoplanin.

Formula $C_{96-97}$ $H_{101-105}$ $O_{35}$ $N_9$ $Cl_2$ Mw about 2027.87

micro-analysis: after drying at 140 °C Δwt 7.1%

| | | | |
|---|---|---|---|
| Calc.: | C 57.45; | H 5.21; | N 6.21 |
| Found: | C 56.6; | H 5.51; | N 6.96 |

b) Preparation of $N^{15}$-(phenylmethoxycarbonyl)teicoplanin $A_2$ cyanomethyl ester (intermediate VI)

Chloroacetonitrile (2 ml) and triethylamine (0.1 ml) are added to a stirred solution of $N^{15}$-CBZ-teicoplanin (intermediate V) in 20 ml of DMF. After 4 hours at room temperature, a second addition of $ClCH_2CN$ and TEA is made and the reaction continued for additional 4 hours. The reaction solvent is evaporated and the residue treated with water and extracted with a mixture n-butanol/ethyl acetate, 3:1. The organic layer is washed with distilled water and concentrated to a small volume. Then ethyl ether (150 ml) is added and a solid separates which is filtered, washed with ether, and dried under reduced pressure at 35 °C, yielding 1.9 g of intermediate VI.
IR (nujol): ν CO 1755 $cm^{-1}$

c) Preparation of $N^{15}$-CBZ-teicoplanin hydrazide (intermediate VII)

98% Hydrazine hydrate (1 ml) or preferably the monoacetate derivative thereof (see example 18) is added to a suspension of $N^{15}$-CBZ-teicoplanin cyanomethyl ester (intermediate VI) (500 ml; 0.2 meq) in 25 ml of absolute ethanol. After about 3 hours at room temperature the reaction is terminated, the solvent is evaporated and the residue is treated with water, collected by filtration and dried under reduced pressure at 40 °C, yielding 450 mg of intermediate VII.

d) Preparation of teicoplanin hydrazide (compound 1) from intermediate VII

Intermediate VII (450 mg) is dissolved in methanol (25 ml) and 0.01 M HCl (1 ml). This solution, after adding 20 mg of 10% Pd/c is hydrogenated at room pressure and temperature.

After 8 hours more than the stoichiometry amount of $H_2$ is adsorbed but only 50% of the CBZ group has been removed. A second portion of 10 mg of 10% Pd/C is added to the reaction mixture and hydrogenation continued to completion (HPLC analysis). The reaction is filtered under reduced pressure on "Celite" and the solvent evaporated under reduced pressure. The residue, treated with ethyl ether, is collected, dried, yielding 380 mg of compound 1.

Example 2: Preparation of teicoplanin aglycon hydrazide, trifluoroacetate (compound 2)

a) Preparation of $N^{15}$-[(1,1-dimethylethoxy)carbonyl] teicoplanin aglycone (intermediate VIII)

Teicoplanin aglycone (7 g; 5.8 meq) is dissolved in 150 ml of DMF at room temperature, and 2,3,4-trichlorophenyl butyl carbonate (2.55 g; 8.5 meq) and TEA (1.1 ml) are added thereto. The reaction mixture is stirred at 40 °C overnight.

The solvent is evaporated under reduced pressure at 50 °C, the obtained residue is dissolved in n-butanol/ethyl acetate, washed with acid water (pH 3) and then twice with distilled water. The solvent is evaporated under reduced pressure and the obtained concentrate (about 30 ml) is diluted with 600 ml of ethyl ether. A solid separates which is collected by filtration and washed with ethyl ether, yielding 5.5 g of intermediate VIII.

b) Preparation of $N^{15}$-[(1,1-dimethylethoxy)carbonyl] teicoplanin aglycon cyanomethyl ester (intermediate IX)

Chloroacetonitrile (1 ml) is added to a solution of $N^{15}$-BOC-teicoplanin aglycone (intermediate VIII) (3 g; 2.3 meq) dissolved in 25 ml of DMF containing $KHCO_3$ (450 mg). The reaction is stirred for about 3 hours at room temperature, then a second portion of $ClCH_2CN$ (0.5 ml) is added and stirring is continued for further 3 hours. The reaction course is controlled by HPLC. When the reaction may be considered terminated (about 6 h) the reaction mass is worked up by diluting it with ethyl ether (200 ml), then collecting the solid which forms washing it with ether and drying it to give 3 g of crude cyanomethyl ester that may be used as such in the next reaction step.
IR (nujol); $\nu$CO, 1755 $cm^{-1}$

c) Preparation of $N^{15}$-BOC-teicoplanin aglycone hydrazide (intermediate X)

The above obtained ester (intermediate IX) (1 g; 0.7 meq) is dissolved in ethanol (30 ml) and (90%) hydrazine hydrate (0.5 ml), a preferably hydrazine monoacetate (see example 18) is added. The reaction is monitored by HPLC and after about one hour at 50°C, it may be considered as completed. The solvent is evaporated and the residue is suspended in water at pH 3 and extracted with n-butanol. The organic layer is washed with distilled water and evaporated; the obtained residue is then treated with ether, recovered by filtration and dried under reduced pressure, yielding 700 mg of intermediate X.

d) Preparation of teicoplanin aglycon hydrazide trifluoro acetate (compound 2) from intermediate X.

Trifluoroacetic acid (4 ml) is added to a suspension of $N^{15}$-BOC-teicoplanin aglycon hydrazide (intermediate X) (1.2 g; 0.9 meq) in $CH_2Cl_2$ (20 ml). The obtained solution is stirred 15 minutes at room temperature then under stirring, ethyl ether is added (150 ml). The solid which forms is collected by filtration, washed with ether and dried under reduced pressure at 50°C, yielding 1.1 g of teicoplanin aglycone hydrazide trifluoroacetate.

Example 3: Preparation of teicoplanin aglycon 1-methyl hydrazide trifluoroacetate (compound 3)

a) Preparation of $N^{15}$-[(1,1-Dimethylethoxy)carbonyl] teicoplanin aglycone 1-methylhydrazide (intermediate XI)

Intermediate IX (1 g; 0.7 meq) is dissolved in absolute ethanol (80 ml) and methyl hydrazine (1 ml) is added thereto. The reaction mixture is stirred at 40°C for 1 hour, then a further portion of methyl hydrazine (1 ml) is added and stirring is continued for 1 hour. The mixture is then concentrated to about 10 ml under reduced pressure and ethyl ether (100 ml) is added thereto. The solid which forms is filtered, dissolved in the minimum amount of $H_2O/CH_3CN$, 1:1 (v/v) and chromatographed on a column packed with 150 g of silanized silica gel (0.063-0.2 mm) Merck CO. in $H_2O/CH_2CN$, 85:15 (v/v). The column is developed with a linear gradient from 15% to 35% $CH_3CN$ in water. The eluted fractions are analyzed by HPLC and those containing pure intermediate XI are pooled; n-butanol is added thereto and evaporation under reduced pressure is continued until both $CH_3CN$ and water are completely evaporated. Then, ether is added and the solid which forms is collected by filtration, washed with ethyl ether and dried at 40°C under pressure. Yield 320 mg of intermediate XI.

b) Prepration of teicoplanin aglycone 1-methyl hydrazide trifluoracetate (compound 3) from intermediate XI

Intermediate XI (300 mg) is hydrolized substantially following the procedure described for intermediate X (see example 2d). After drying at 40°C under reduced pressure, 320 mg of title compound are obtained.
NMR ($DMSOd_6$) $\delta$ ; 2.8 (s, $CH_3$)

<u>Example 4</u>: Preparation of teicoplanin aglycon 1-ethyl hydrazide trifluoroacetate (compound 4)

a) Preparation of N[15]-tBOC teicoplanin aglycon 1-ethyl hydrazide (intermediate XII)

To a solution of intermediate VIII (1 g; 0.77 mg) in DMF (50 ml) ethyl hydrazine oxalate (115 mg, 1.15 meq) is added. The solution is cooled to 0 °C and diethylphosphoryl cyanide (DEPC) (0.2 ml; 1.15 mmole) and triethyl amine (0.3 mg; 2.3 meq) are added. The reaction mixture is stirred at 5 °C for 1 hour, then 4 hours at room tempeature. If HPLC analysis shows that about 50% of the teicoplanin starting material is present, the same amounts of the other reagents as used above is added again at room temperature and the reaction mixture stirred overnight. After this time, HPLC analysis shows no more than 20% of the teicoplanin starting material. The solvent is evaporated at 50 °C (external temperature) under reduced pressure. The residue is purified on a chromatographic column as described for intermediate XI (see example 3a). Yield 250 mg of inermediate XII.

b) Preparation of teicoplanin aglycon 1-ethyl hydrazide trifluoroacetate (compound 4) from interemediate XII.

Intermediate XII (200 mg) is hydrolized according to substantially the same procedure as described for the preparation of compound 3 from intermediate XI (see example 3)
Yield: 180 mg of the title compound.
NMR (DMSO$d_6$)$\delta$ : 1.09 (t, $CH_3$); 2.97 (q, $CH_2$)
p$K_{mcs}$ 6.6
U.V. ($\lambda$max, nm) 279 (phosphate buffer pH 7.4)

Example 5: Preparation of teicoplanin aglycon 2-benzylhydrazide trifluoroacetate (compound 5)

To a solution of intermediate X (1.5 g; 1.1 meq) in a mixture of $CH_3COOH$ (22 ml) and MeOH (11 ml), benzaldehyde (1.5 Ml) and a catalytic amount of p-toluensulphonic acid (1 mg) are added. After about 15 minutes of stirring at room temperature the HPLC analysis (gradient profile 2) shows a peak at $t_R$ 25.5 min (2-benzylidenehydrazide derivative). Two portions of $NaCNBH_3$ (0.5 g each) are then added with a 20 min interval. The compound with $t_R$ 25.5 min completely transforms in a compound having $t_R$ 24.2 min. The solvent is evaporated under reduced pressure, then the residue is treated with distilled water (50 ml) and the solid which forms is filtered. The wet crude material is suspended in $CH_3CN$:$H_2O$ 70:30 and contacted with silanized silica-gel (0.06-0.2 mm; Merck Co.) (10 g); after removing the solvent under reduced pressure the solid is loaded on a chromatographic column containing 120 g of the same silanized silica gel. Impurities are removed by eluting with $CH_3CN$:$H_2O$ 30:70 then the compound having $t_R$ 24 is eluted with a linear gradient from 40% to 60% $CH_3CN$ in water.

Fractions containing the pure compound of the title are pooled, n-butanol is added and $CH_3CN$ and water are completely evaporated under reduced pressure. Then ether is added and the product which forms is filtered, washed with ether and dried overnight at 50 °C under reduced pressure, obtaining 500 mg of intermediate XIII. This compound (450 mg) is suspended in $CH_2Cl_2$ (2 ml) and trifluoroacetic acid (1 ml) is added with stirring. After 15 minutes the reaction mixture is diluted with 100 ml of ethyl ether and the precipitate which forms is collected by filtration, washed with ether and dried under reduced pressure, yielding 420 mg of pure teicoplanin aglycone 2-benzylhydrazide trifluoroacetate.
Potentiometric titration p$K_{mcs}$ 6.55
Formula $C_{65}H_{53}O_{17}N_9Cl_2.2CF_3COOH$ ΔMw 1531.019
Microanalysis: weight loss at 140 °C wt 9.5%

| | | | |
|---|---|---|---|
| Calc. | C 54.1; | H 3.6; | N 8.2 |
| Found | C 54.3; | H 4.2; | N 8.3 |

Example 6: Prepration of teicoplanin aglycon 2-propyl hydrazide ditrifluoroacetate (compound 6)

a) Preparation of N[15]-BOC-aglycon 2-propyl hydrazide

To a solution of intermediate X (700 mg; 0.5 mmol) in a mixture of n-butanol (60 ml), water (5.5 ml) and acetic acid (1.2 ml) propionic aldehyde (0.6 ml, 8.33 mmol) is added. The mixture is stirred 20 minutes at room temperature then a second portion of the aldehyde (0.6 ml) is added and the reaction continued under the same conditions for an hour. HPLC analysis (program 2) shows starting material ($t_R$ 15.6) and Schiff base ($t_R$ 18.78) with a ratio in the range 30:70, respectively.

$NaBH_4$ pellets (1 g) are added under stirring and the reaction temperature is maintained below 45°C, cooling with a water bath. When all the $NaBH_4$ is dissolved and the internal temperature is about 25°C, propionic aldehyde (0.6 ml) is added and the reaction continued for one hour, then $NaBH_4$ (0.5 g) is added and stirring is continued until disappearance of the starting material. The reaction mixture is then acidified by adding diluted hydrochloric acid. The precipitate which forms is collected by filtration, dissolved in water/acetonitrile 70/30, loaded on a column of silica gel and eluted with 1 l of water/$CH_3CN$ 70/30 and 1 l of water/$CH_3CN$, 63/37. Yield 330 mg of $N^{15}$-BOC-aglycon 2-propyl hydrazide (intermediate XIV).

b) This product is hydrolized with $CF_3COOH/CH_2CH_2$. yielding 200 mg of title compound.

$pk_{mcs}$ 6.45
Formula: $C_{61}H_{53}O_{17}N_9Cl_2.2CF_3COOH$ MW 1483.06
microanalysis: weight loss at 140°C 4.3%

| | | | | |
|---|---|---|---|---|
| Calc: | C 52.5; | H 3.7; | N 8.5; | Cl 4.8 |
| Found | C 51.34; | H 4.3 | N 8.6; | Cl 4.4 |

Example 7: Preparation of teicoplanin 2-(2-dimethylamino)ethyl hydrazide trifluoroacetate (compound 7)

a) Preparation of $N^{15}$-Boc-teicoplanin aglycon 2-(2-dimethylamino)ethyl hydrazide (intermediate XV)

To a solution of intermediate X (1.2 g; 0.9 mmol) in n-butanol (80 ml) and water (10 ml), dimethylamino acetaldehyde hydrochloride obtained from dimethylacetaldehyde ethyl acetate (Aldrich) by hydrolysis with HCl according to J.H. Biel, W.K. Hoya and H.A. Leiser, J.A.C.S. 81, 2527; 1959 (200 mg; 1.36 mmol) and sodium acetate (246 mg; 3 mmol) are added under nitrogen atmosphere. The mixture is stirred 1 hour at room temperature then $NaCNBH_3$ (30 Mg; 0.4 mmol) is added. After 2 hours a second portion of aldehyde (200 mg) is added followed after 1 hour by NaCNBH (30 mg). The reaction is continued overnight. The pH is adjusted to pH 3 with N HCl and the solvent evaporated under reduced pressire. The residue is dissolved in $CH_3CN/H_2O$ 30/70 and purified on a chromatographic column as described for intermediate XI, yielding 500 mg of the title compound.

b) Preparation of teicoplanin aglycone 2-(2-dimethylamino)ethyl hydrazide trifluoroacetate - Compound 6

Trifluoro acetic acid (1.5 ml) is added to a suspension of $N^{15}$-Boc-teicoplanin aglycone 2-(2-dimethylamino)ethyl hydrazide (intermediate XV) (500 mg; 0.36 mmol) in $CH_2Cl_2$ and it is hydrolized substantially following the procedure described for compound 2 (see example 2d). After drying at 40°C under reduced pressure 470 mg of compound 7 are obained.
NMR ($MDSOd_6$) 2.79 (S, $CH_3$); 3.4-3.6 (t, $CH_2CH_2$-N)
$pk_{mcs}$ 6.55, 7.9

Example 8: Preparation of teicoplanin 2-cyclopentyl hydrazide (compound 8)

To a solution of $N^{15}$ CBz teicoplanin hydrazide (intermediate VII) (1 g, 0.5 mmol) in 50 ml MeOH and 25 ml of 0.005 HCl, cyclopentenone (1 g, 11.9 mmol) is added and the mixture stirred at room temperature for 1 hours. The Schiff's base is enought stable to be analyzed by HPLC ($t_R$ 21.6 gradient /2 7 ). 5% Pd/C (1 g) is added under nitrogen and the mixture hydrogenated at room pressure for 40 minutes (consumed 50

ml of $H_2$). The catalyst is filtered on "Celite" (filter aid) and the solvent evaporated. The residue is purified on a chromatographic column of silanized silica gel.
Yield 300 mg NMR (DMSOd$_6$) 1.44-1.70 (m 4CH$_2$); 3.69 (m CH);

Example 9: Preparation of teicoplanin aglycon 2,2-dipropyl hydrazide trifluoroacetate (compound 9)

To a solution of intermediate X (1.2 g; 0.9 mmol) in MeOH (30 ml) and acetic acid (15 ml), propionaldehyde (1.5 ml; 20.7 mmol) and p.toluensulfonic acid (1.2 mg) are added. The mixture is stirred at room temperature for 30 minutes, then $NaCNBH_3$ (0.5 g; 7.9 mmol) is added. After about 2 h of reaction, adjusted to about 2 adding N HCl and the solvent is evaporated under reduced pressure. The residue, dissolved in a mixture $CH_3CN/H_2O$ 20/30, is chromatographed through a 150 g column of silanized silica gel eluting with 25% aqueous $CH_3CN$ (500 ml) and then with a linear gradient from 25% to 40% of $CH_3CN$ in water. Fractions containing the pure intermediate XVI are pooled, evaporated and treated with ethyl ether. The precipitate is collected and dried at 50° C under reduced pressure, yielding 550 mg of intermediate XVI ($N^{15}$-BOC-teicoplanin aglycon 2,2-dipropyl hydrazide.

The BOC protecting group is removed from it with trifluoroacetic acid in $CH_2Cl_2$ as described above obtaining 500 mg of compound 9.
$pk_{mcs}$ 6.5
FAB: $M^+$ 1297
Microanalysis: weight loss at 140° C = 4.3%

| | | | |
|---|---|---|---|
| Calc: | C 53.5; | H 4.0; | N 8.2 |
| Found | C 52.9; | H 4.4; | N 8.1 |

Example 10: Preparation of teicoplanin aglycone 1-methyl-2-propyl hydrazide trifluoroacetate (compound 10)

To $N^{15}$-Boc teicoplanin aglycone 1-methyl hydrazide (intermediate XI) (2 g; 1.5 mmol) dissolved in MeOH (60 ml) propionaldehyde (1.5 ml; 20.7 mmol) and p.toluensulfonic acid (15 mg) are added and the mixture stirred at room temperature for 2 h. Then $NaBH_4$ (pellets; 100 mg) is added and the reaction is continued overnight. The solvent is evaporated under reduced pressure and the residue is suspended in 40 ml of water, pH 4, and purified by column chromatography as above reported, yielding 320 mg of intermediate XVII ($N^{15}$-BOC-teicoplanin 1-methyl-2-propyl hydrazide).

Removing the BOC group from intermediate XVII with trifluoroacetic acid/$CH_2Cl_2$, according to the procedure reported above, give 300 mg of the title compound.

Example 11: Preparation of teicoplanin aglycon 2,2-dimethyl hydrazide trifluoroacetate (compound 11)

To a solution of intermediate X (1.2 g; 0.9 mmol) in $CH_3CN$ (72 ml) and water (9.6 ml); 40% aq. formaldehyde (1.2 ml) is added. The solution is stirred at room temperature for 15 minutes then two portions of $NaCNBH_3$ (100 mg) are added at 2 h intervals. The reaction is continued overnight then the organic solvent is evaporated under reduced pressure and the residue diluted with water (50 ml). The pH is adjusted to 3 and the mixture is extracted with n-butanol (100 ml). The organic layer is separated, washed with water (30 ml) and evaporated under reduced pressure. The residue is suspended in ethyl acetate, collected by filtration, washed with ethyl ether and dried under reduced pressure, yielding 800 mg of intermediate XVIII ($N^{15}$-BOC-teicoplanin aglycon 2,2-dimethyl hydrazide), whose protecting group is removed as usual with trifluoroacetic acid/methylene chloride, yielding 750 mg of the title compound.
Formula $C_{60}H_{51}O_{17}N_9Cl_2.CF_3COOH$ Mw 1355
Microanalysis: weight loss at 140° C 5.3%

| | | | | |
|---|---|---|---|---|
| Calc. | C 54.9; | H 3.9; | N 9.3; | Cl 5.2 |
| Found | C 54.6; | H 4.7; | N 9.9; | Cl 5.0 |

Example 12: Preparation of teicoplanin aglycone 2-(2,3-dihydroxy)propyl hydrazide trifluoroacetate (compound 12)

To a solution of intermediate X (1.2 g; 0.9 mmol) in n-butanol (120 ml) and water (100 ml), D,L-glyceraldehyde (600 mg, 6.6 mmol) and p.toluenesulfonic acid (60 mg) are added, with stirring at room temperature for 20 min. $NaCNBH_3$ (300 mg; 127 mmol) is then added and the reaction continued. After two hours, a second portion of D,L-glyceraldehyde (600 mg) is added followed, after 30 min, by $NaCNBH_3$ (700 mg). The reaction is continued for 3 hours, then the pH is adjusted to 8 with $NaHCO_3$, ethyl acetate (50 ml) is added and the mixture is washed with water (40 ml x 3 times), the organic solvent is evaporated under reduced pressure and the residue is mixed with Celite filter aid and loaded on a chromatographic column prepared with 150 g of silanized silicagel in $CH_3CN/H_2O$ 10/90. This column is eluted with with 1 l of 30% aqueous $CH_3CN$ and then 1 l of 40% aqueous $CH_3CN$. By collecting the fractions containing intermediate XIX ($N^{15}$-BOC-teicoplanin aglycon 2-(2,3-dihydroxy propyl hydrazide) and working them up as described in the previous examples, 600 mg of intermediate XIX is recovered.

Removing the BOC group from it according to the method above reported $CF_3COOH/CH_2Cl_2$ the title compound (450 mg) is obtained.

$pk_{mcs}$ 6.7

Formula $C_{61}H_{53}O_{19}N_9Cl_2.2CF_3COOH$ Mw 1515-04

Microanalysis: weight loss at 140 °C = 11.8%, residue 2%

| | | | | |
|---|---|---|---|---|
| Calc. | C 51.53; | H 3.65; | N 8.31; | Cl 4.67 |
| Found | C 51.15; | H 4.07; | N 8.17; | Cl 4.38 |

Example 13: Preparation of teicoplanin aglycone 2-(2-butenylidene) hydrazide trifluoroacetate (compound 13)

To a solution of intermediate X (1.2 g; 0.9 mmol) in $CH_3CN$ (72 ml) and water (10 ml) crotonaldehyde (0.6 ml; 7.3 mmol) is added. The solution is stirred at room temperature for 15 minutes, then $NaCNBH_3$ - (100 mg) is added. A second portion of $NaCNBH_3$ (100 mg) is added after 1 hour and the reaction is continued for further 3 hours. The solvent is evaporated, water (80 ml) is added to the residue, the resulting mixture is adjusted to pH 3 with 1N hydrochloric acid and extracted with 200 ml of n-butanol/ethylacetate 3:1. The organic layer is washed with 30 ml of water and evaporated under reduced pressure. The residue is suspended in ethyl ether, collected by filtration and dried under reduced pressure, yielding 1.2 g of intermediate XX ($N^{15}$-BOC-teicoplanin aglycon 2-(2-butenylidene)hydrazide) which is then treated with trifluoroacetic acid/methylene chloride to remove the BOC protecting group as described above to give compound 13 (1.1 g).

Example 14: Preparation of teicoplanin aglycon 2-cyclopentyl hydrazide trifluoroacetate (compound 14)

To a solution of intermediate X (1.2 g; 0.9 mmol) in n-butanol (60 ml) and water (10 ml), cyclopentanone (1 ml; 11.3 mmol) and p.toluensulfonic acid (25 mg) are added. The mixture is stirred at room temperature for 1 hour. The HPLC analysis (gradient profile 2) shows the formation of the Schiff's base ($t_R$ 19.5; $N^{15}$-Boc teicoplanin aglycone 2-cyclopentylidene hydrazide). $NaCNBH_3$ (1 g) is added in small portions during 1 hour. A second portion of cyclopentanone (0.2 ml) is added followed after 30 min by $NaCNBH_3$ (100 mg). The reaction is continued for 3 hours. The solvent is then evaporated under reduced pressure and the residue is chromatographed on a silanized silica gel column eluting with a gradient from 25% to 40% of $CH_3CN$ in water. The fractions containing intermediate XXI (HPLC analysis) are combined, concentrated and the residue is suspended in ethyl ether, and collected by filtratión to give 600 mg of intermediate XXI ($N^{15}$-

BOC-teicoplanin aglycon 2-cyclopentyl hydrazide). This product is treated with trifluroacetic acid/methylene chloride to remove the BOC group, yielding 500 mg of the title compound.

Example 15: Preparation of teicoplanin aglycone 2-[(1,3-dimethyl)butyl]hydrazide trifluoroacetate (compound 15)

To a solution of intermediate X (1.2 g; 0.9 mmol) in n-butanol (108 ml) and water (12 ml) methyl isobutyl ketone (1.2 ml, 9.6 mmol) is added. The solution is stirred at room temperature for 1 hour, then $NaCNBH_3$ (0.8 g) is added. After 2 hours, the solution is diluted with ethyl acetate (30 ml) and washed with water (50 ml). The organic phase is evaporated under reduced pressure and the residue is suspended in 10 ml of ethyl acetate and 100 ml of ether. The precipitate is collected by filtration and dried under reduced pressure yielding 0.9 g of intermediate XXII ($N^{15}$BOC-teicoplanin aglycone 2-[(1,3-dimethyl)butyl]hydrazide) which is then treated as above reported with 2 ml of $CH_2Cl_2$ and 2 ml of $CF_3COOH$ at room temperature for 15 min. The solvents are evaporated and the residue taken up with ether. The precipitate is collected by filtration and dried under reduced pressure to give 650 mg of the title compound.

Example 16: Preparation of teicoplanin aglycone 2-(1-phenylethylidene)hydrazide trifluoroacetate (compound 16)

To a solution of intermediate X (1.2 g; 0.9 mmol) in n.butanol (140 ml) and water (12 ml), acetophenone (1.2 ml; 10 mmol) is added and the solution is allowed to stand for 2 hours at room temperature. The solvent is evaporated under reduced pressure and the residue is purified on a silica gel column eluting with absolute EtOH. The fractions containing intermediate XXIII are combined, concentrated under reduced pressure, the residue is taken up with ether, collected by filtration and dried under reduced pressure to give 0.9 mg of intermediate XXIII ($N^{15}$-BOC-teicoplanin aglycon 2-(1-phenylethylidene)hydrazide).

This product is suspended in 2 ml of $CH_2Cl_2$ and treated with $CF_3COOH$ (2 ml) at room temperature. After 1.5 hours the solvent is evaporated, and the residue is diluted with ethyl acetate which is then evaporated. This residue is suspended in ethyl ether and the obtained precipitate is collected by filtration and dried to give 750 mg of the title compound.

Example 17: Preparation of teicoplanin aglycon 2-(2-aminoethyl)hydrazide trifluoroacetate (compound 17)

To a solution of interemediate X (1.15 g; 0.87 mmol) in DMF (70 ml), 2-bromoethylamine hydrobromide (0.2 g; 0.9 mmol) is added. The reaction is heated at 50° for 4 hours then a second portion of n 2-bnromoethylamine hydrobromide (0.2 g) is added followed after 2 hours by triethylamine (150 ml; 1.07 mmol). Reaction is continued over night at room temperature. HPLC analysis shows a 70% transformation. The solvent is evaporated under vacuum, and the residue dissolved in $CH_3CN$/water, 30/70 is chromatographed on silanized silica gel (100 g) eluted with a linear gradient from 20% to 35% of $CH_3CN$ in water. The pure fractions containing intermediate XXIV are pooled and evaporated and the residue, treated with ether, is collected and dried to give 350 mg of intermediate XXIV ($N^{15}$-BOC-teicoplanin aglycon 2-(2-aminoethyl)-hydrazide).

Formula $C_{65}H_{60}O_{19}N_{10}Cl_2$ Mw 1356

FAB 1350

Intermediate XXIV (300 mg) is dissolved in a mixture $CF_3COOH$ $CH_2Cl_2$, 1:1 (3 ml) and the solution stirred at room temperature for 40 minutes. The reaction is diluted with ether (60 ml) and stored in refrigerator for 3 hours then the precipitate which forms collected, washed with ether and dried to give 240 mg of the compound of the title.

Example 18:

Alternative preparation of $N^{15}$-BOC-teicoplanin aglycon hydrazide (intermediate X)

To a solution of $N^{15}$-BOC-teicoplanin aglycon cyanomethyl ester (intermediate IX) (10 g, 7.55 mmol) in

500 ml of absolute ethanol, acetic acid (4.3 ml; 75 mmol) and hydrazine monohydrate (3.7 ml, 76 mmol) are added. The reaction mixture is stirred 12 hours at room temperature, then acetic acid (0.21 ml) and hydrazine (0.185 ml) are added again and the reaction continued for 3 hours. The solvent is evaporated under reduced pressure; the residue, diluted with water (200 ml) and the pH corrected to 3 with diluted hydrochloric acid, is extracted with 500 ml butanol and 200 ml ethyl acetate. The organic layer, washed three times with water, is evaporated to 60 ml and diluted with 350 ml of ether chilling the mixture for about one hour, is filtered, the precipitate is collected by filtration, washed with ether and dried under reduced pressure. Yield 8.2 of pure title compound.

## Claims

1. New teicoplanin hydrazides of formula I

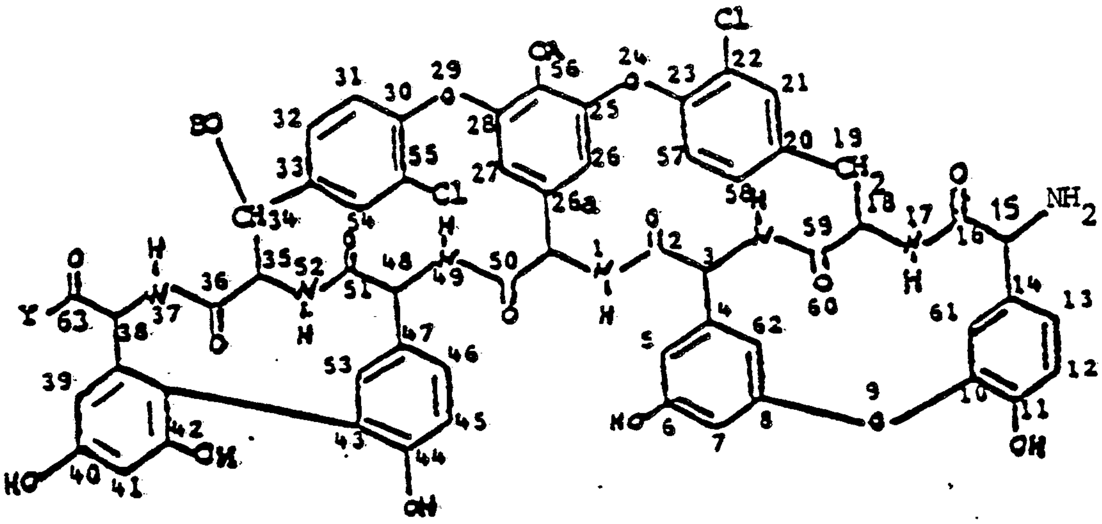

wherein

Y represents a group $R^1R^2N$-NR- wherein

R represents hydrogen or $(C_1$-$C_4)$alkyl,

$R^1$ represents hydrogen, $(C_1$-$C_6)$alkyl, optionally substituted with 1 or 2 groups selected from hydroxy, amino, $(C_1$-$C_4)$alkylamino, di$(C_1$-$C_4)$alkylamino, a nitrogen containing 5-6 membered saturated heterocyclic ring, $(C_1$-$C_4)$alkoxy, $(C_2$-$C_6)$alkenyl, $(C_5$-$C_7)$cycloalkyl, phenyl optionally substituted with a group selected from chloro, bromo, fluoro, iodo, $(C_1$-$C_4)$alkyl and hydroxy,

$R^2$ represents hydrogen or $(C_1$-$C_4)$alkyl, or

$R^1$ and $R^2$ taken together with the adjacent nitrogen atom represent a 5-6 membered saturated heterocyclic ring or a a group of formula $=CR^3R^4$ wherein $R^3$ represents hydrogen or methyl and $R^4$ represents $(C_1$-$C_4)$alkyl or phenyl,

A represents hydrogen or -N[$(C_{10}$-$C_{11})$aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alfa-D-mannopyranosyl;

with the proviso that B represents hydrogen only when A and M are simultaneously hydrogen and M represents hydrogen only when A is hydrogen

and the addition salts thereof.

2. A compound according to claim 1 wherein A, B and M either represent the sugar moieties defined in claim 1 or represent hydrogen atoms.

3. A compound according to claim 1 wherein A and M represent the sugar moieties defined above and A represents N-(8-methylnonanoyl)-beta-D-2-deoxy-2-amino-glucopyranosyl.

4. A compound according to claim 1 wherein R represents hydrogen or methyl, $R^2$ represents hydrogen or ($C_1$-$C_4$)alkyl and $R^1$ represents benzyl, mono- or di-($C_1$-$C_4$)alkylamino($C_1$-$C_4$)alkyl, ($C_1$-$C_4$)alkyl, ($C_5$-$C_6$)-cycloalkyl, mono- or di-hydroxy($C_1$-$C_4$)alkyl, ($C_2$-$C_4$)alkynyl, and amino($C_1$-$C_4$)alkyl, or $R^1$ and $R^2$ taken together with the adjacent nitrogen atom represent pyrrolidinyl, piperidinyl, 4-methylpiperidinyl, piperazinyl, 4-methylpiperazinyl and morpholinyl.

5. A compound according to claim 1 wherein B and M either represent the sugar moieties defined above or represent hydrogen atoms or B and M represent the sugar moieties defined above, A represents hydrogen or N-(8-methylnonanoyl)-beta-D-2-deoxy-2-amino-glucopyranosyl and R, $R^1$ and $R^2$ have the meanings reported below:

| R | $R^1$ | $R^2$ |
|---|---|---|
| H | H | H |
| $CH_3$ | H | H |
| $C_2H_5$ | H | H |
| H | $PhCH_2$ | $CH_3$ |
| H | $(CH_3)_2NCH_2CH_2$ | H |
| H | $CH_3CH_2CH_2$ | H |
| H | cyclopentyl | H |
| $CH_3$ | $CH_2CH_2N(CH_3)_2$ | H |
| H | $-CH(CH_3)CH_2N(CH_3)_2$ | H |
| $CH_3$ | $CH_2CH_2NH_2$ | H |
| H | $CH_2CH_2NH_2$ | $CH_3$ |
| H | $CH_2CH_2N$ 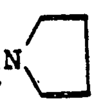 | H |
| H | $CH_2CH_2N$ 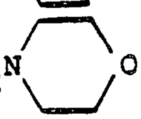 | H |
| H | $CH(CH_3)CH(CH_3)CH_2N(CH_3)_2$ | H |
| H | $CH_2CH_2NH-$ 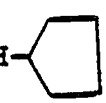 | H |

6. A process for preparing a compound of claim 1 to 5 which comprises reacting a compound of formula

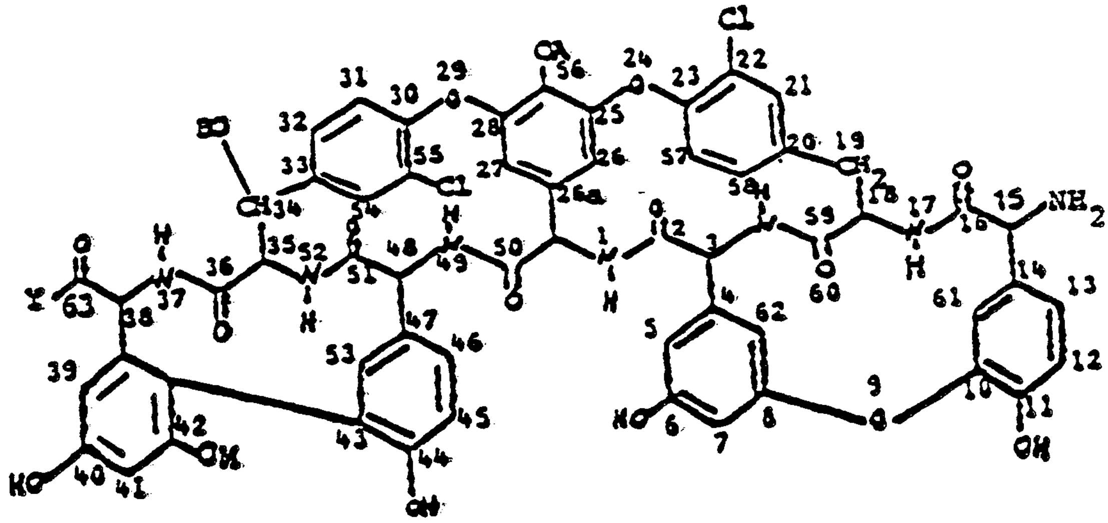

wherein Y is hydroxy, A represents hydrogen or -N[($C_{10}$-$C_{11}$)aliphatic acyl]-beta-D-2-deoxy -2-amino-glucopyranosyl, B represents hydrogen or N-acetyl-beta-D-2-deoxy-2-amino-glucopyranosyl, M represents hydrogen or alfa-mannopyranosyl, with the proviso that B may represent hydrogen only when A and M are simultaneously hydrogen and M may represent hydrogen only when A is hydrogen, a salt thereof, or a mixture thereof in any proportion, with a hydrazine derivative of formula $R^1R^2NNHR$ wherein R, $R^1$, and $R^2$ are as defined above, in a polar aprotic solvent in the presence of a condensing agent.

7. A process according to claim 5 wherein the condensing agent is selected from ($C_1$-$C_4$)alkyl, phenyl or heterocyclic phosphorazidates.

8. A process according to claim 5 wherein the condensing agent is selected from diphenyl phosphorazidate, diethyl phosphorazidate, di(4-nitrophenyl)phosphorazidate, dimorpholylphosphorazidate, diphenylphosphorochloridate and diethylphosphoryl cyanide.

9. A process according to claim 5 wherein the polar aprotic solvent is selected from aliphatic amides, alkyl ethers, ethers of glycols and polyols, phosphoramides, sulfoxides, cyclic substituted ureas and aromatic solvents.

10. A process according to claim 5 wherein the starting material is protected in position 15 with an amino protecting group.

11. A compound of claims 1 to 5 for use as a medicine.

12. Use of a compound of claims 1 to 5 for preparing a medicament for antibiotic use.

13. A pharmaceutical composition comprising a compound of claim 1 in admixture with a pharmaceutically acceptable vehicle.

Claims for the following Contracting States: GR, ES

1. A process for preparing new teicoplanin hydrazides of formula I

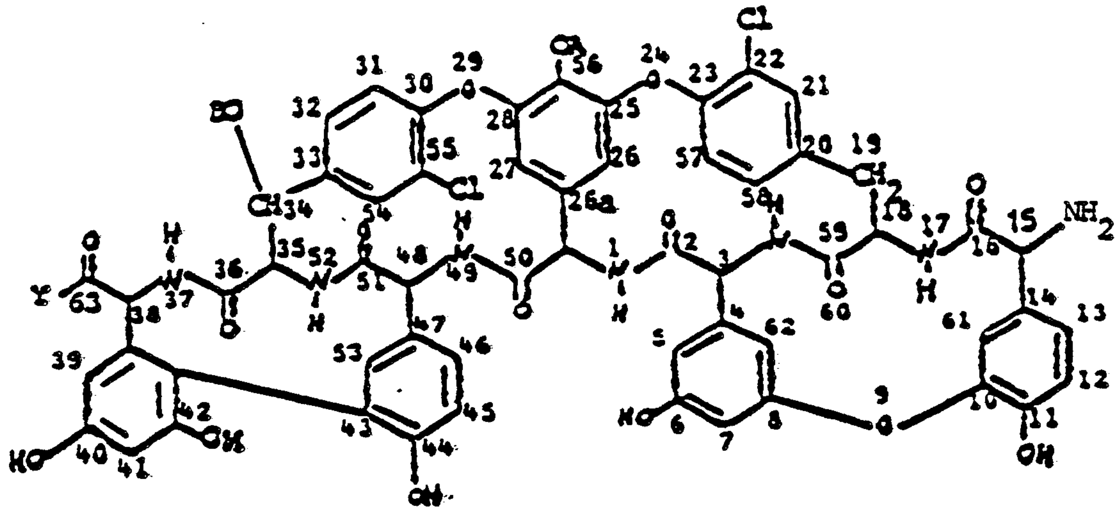

wherein

Y represents a group $R^1R^2N$-NR- wherein

R represents hydrogen or ($C_1$-$C_4$)alkyl,

$R^1$ represents hydrogen, ($C_1$-$C_6$)alkyl, optionally substituted with 1 or 2 groups selected from hydroxy, amino, ($C_1$-$C_4$)alkylamino, di($C_1$-$C_4$)alkylamino, a nitrogen containing 5-6 membered saturated heterocyclic ring, ($C_1$-$C_4$)alkoxy, ($C_2$-$C_6$)alkenyl, ($C_5$-$C_7$)cycloalkyl, phenyl optionally substituted with a group selected from chloro, bromo, fluoro, iodo, ($C_1$-$C_4$)alkyl and hydroxy,

$R^2$ represents hydrogen or ($C_1$-$C_4$)alkyl, or

$R^1$ and $R^2$ taken together with the adjacent nitrogen atom represent a 5-6 membered saturated heterocyclic ring or a a group of formula $=CR^3R^4$ wherein $R^3$ represents hydrogen or methyl and $R^4$ represents ($C_1$-$C_4$)alkyl or phenyl,

A represents hydrogen or -N[($C_{10}$-$C_{11}$)aliphatic acyl]-beta-D-2-deoxy-2-amino-glucopyranosyl,

B represents hydrogen or N-acetyl-beta-D-2-deoxy-2-amino-glucopyranosyl,

M represents hydrogen or alfa-D-mannopyranosyl;

with the proviso that B represents hydrogen only when A and M are simultaneously hydrogen and M represents hydrogen only when A is hydrogen

and the addition salts thereof, which comprises reacting a compound of formula

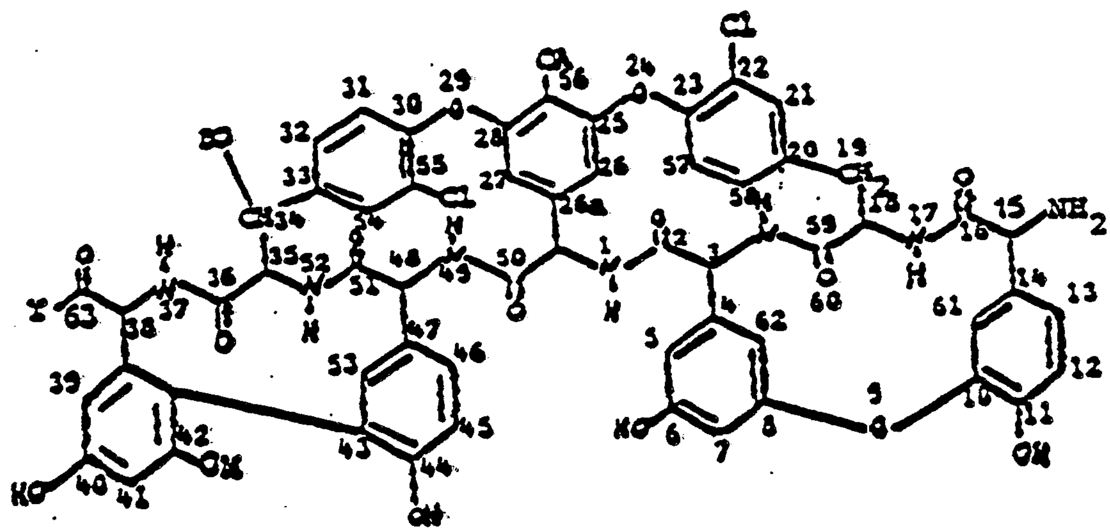

wherein Y is hydroxy, A represents hydrogen or -N[($C_{10}$-$C_{11}$)aliphatic acyl]-beta-D-2-deoxy-2-amino-glucopyranosyl, B represents hydrogen or N-acetyl-beta-D-2-deoxy-2-amino-glucopyranosyl, M represents hydrogen or alfa-mannopyranosyl, with the proviso that B may represent hydrogen only when A and M are simultaneously hydrogen and M may represent hydrogen only when A is hydrogen, a salt thereof, or a

mixture thereof in any proportion, with a hydrazine derivative of formula $R^1R^2NNHR$ wherein R, $R^1$, and $R^2$ are as defined above, in a polar aprotic solvent in the presence of a condensing agent.

2. A process according to claim 1 for preparing a compound of formula I wherein A, B and M either represent the sugar moieties defined in claim 1 or represent hydrogen atoms.

3. A process according to claim 1 for preparing a compound of formula I wherein A and M represent the sugar moieties defined above and A represents N-(8-methylnonanoyl)-beta-D-2-deoxy-2-amino-glucopyranosyl.

4. A process according to claim 1 for preparing a compound of formula I wherein R represents hydrogen or methyl, $R^2$ represents hydrogen or $(C_1\text{-}C_4)$alkyl and $R^1$ represents benzyl, mono- or di-$(C_1\text{-}C_4)$-alkylamino$(C_1\text{-}C_4)$alkyl, $(C_1\text{-}C_4)$alkyl, $(C_5\text{-}C_6)$cycloalkyl, mono- or di-hydroxy$(C_1\text{-}C_4)$alkyl, $(C_2\text{-}C_4)$alkynyl, and amino$(C_1\text{-}C_4)$alkyl, or $R^1$ and $R^2$ taken together with the adjacent nitrogen atom represent pyrrolidinyl, piperidinyl, 4-methylpiperidinyl, piperazinyl, 4-methylpiperazinyl and morpholinyl.

5. A process according to claim 1 for preparing a compound of claim 1 wherein B and M either represent the sugar moieties defined above or represent hydrogen atoms or B and M represent the sugar moieties defined above, A represents hydrogen or N-(8-methylnonanoyl)-beta-D-2-deoxy-2-amino-glucopyranosyl and R, $R^1$ and $R^2$ have the meanings reported below:

| R | $R^1$ | $R^2$ |
|---|---|---|
| H | H | H |
| $CH_3$ | H | H |
| $C_2H_5$ | H | H |
| H | $PhCH_2$ | $CH_3$ |
| H | $(CH_3)_2NCH_2CH_2$ | H |
| H | $CH_3CH_2CH_2$ | H |
| H | cyclopentyl | H |
| $CH_3$ | $CH_2CH_2N(CH_3)_2$ | H |
| H | $-CH(CH_3)CH_2N(CH_3)_2$ | H |
| $CH_3$ | $CH_2CH_2NH_2$ | H |
| H | $CH_2CH_2NH_2$ | $CH_3$ |
| H | $CH_2CH_2$ 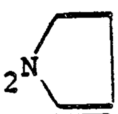 | H |
| H | $CH_2CH_2$ 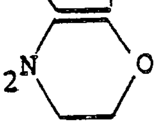 | H |
| H | $CH(CH_3)CH(CH_3)CH_2N(CH_3)_2$ | H |
| H | $CH_2CH_2NH-$ 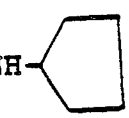 | H |

6. A process for preparing a compound according to claims 1, 2, 3, 4 or 5 wherein the condensing agent is selected from $(C_1\text{-}C_4)$alkyl, phenyl or heterocyclic phosphorazidates.

7. A process for preparing a compound according to claims 1, 2, 3, 4, 5 or 6 wherein the condensing agent is selected from diphenyl phosphorazidate, diethyl phosphorazidate, di(4-nitrophenyl)phosphorazidate, dimorpholylphosphorazidate, diphenylphosphorochloridate and diethylphosphoryl cyanide.

8. A process for preparing a compound according to claims 1, 2, 3, 4, 5, 6 or 7 wherein the wherein the polar aprotic solvent is selected from aliphatic amides, alkyl ethers, ethers of glycols and polyols, phosphoramides, sulfoxides, cyclic substituted ureas and aromatic solvents.

9. A process for preapring a compound according to claim 1, 2, 3, 4, 5, 6, 7 or 8 wherein the starting material is protected in position 15 with an amino protecting group.

10. Use of a compound of claims 1 to 9 for preparing a medicament for antibiotic use.